# EUROPEAN PATENT APPLICATION

(11) **EP 4 800 030 A1**
(43) Date of publication of application: **02.09.2026**
(21) Application number: 25893265.6
(22) Date of filing: 22.12.2025
(51) Int. Cl.: C07K 16/08, G01N 33/569, G01N 33/577, A61K 39/42, A61P 31/22, A61P 35/00, C12N 15/13

(54) **MONOCLONAL ANTIBODY FAB5 RECOGNIZING GB PROTEIN OF EB VIRUSES AND USE THEREOF**

(30) Priority: 23.12.2024 CN 202411904883
(71) Applicant: Sun Yat-Sen University Cancer Center (SYSUCC), Guangzhou Guangdong 510060 (CN)
(72) Inventor: ZENG, Musheng, Guangzhou, Guangdong 510060 (CN); SUN, Cong, Guangzhou, Guangdong 510060 (CN); XIE, Chu, Guangzhou, Guangdong 510060 (CN)
(74) Representative: Gassner, Birgitta
(86) International application number: PCT/CN2025/144285
(87) International publication number: WO 2026/138729

(57) **Abstract**

The present disclosure belongs to the technical field of antibody, and discloses a monoclonal antibody Fab5 recognizing EBV gB protein and use thereof. The monoclonal antibody or the antigen-binding fragment thereof has high affinity for gB protein and can significantly inhibit EBV infection of epithelial cells and B cells, and the epitope of Fab5 binding to gB is determined through structural analysis. The antibody can be used for detecting a presence or level of gB protein in a sample, detecting EBV, diagnosing a disease caused by EBV infection, and preventing EBV infection and/or treating and/or preventing a disease caused by EBV infection.

## Description

### TECHNICAL FIELD

The present disclosure belongs to the technical field of antibody, and specifically relates to a monoclonal antibody Fab5 recognizing Epstein-Barr virus gB protein and use thereof.

### BACKGROUND

Epstein-Barr virus (EBV), also known as human herpesvirus type 4, was first established in 1964 by Epstein and Barr by culturing Burkitt's lymphoma cells *in vitro* through suspension culture. EBV is a DNA oncogenic virus of the gamma subfamily of lymphotropic viruses. EBV particle consists of four structural components: core protein, capsid, tegument, and envelope. The core is a DNA-wrapped core protein.

EBV most commonly establishes a latent infection, about 90% or more of EBV-infected individuals develop lifelong latent infection, and the virus is activated under certain conditions to initiate carcinogenesis. Current research has found that human EBV is closely associated with the occurrence of malignant tumors such as nasopharyngeal carcinoma, infectious mononucleosis, Hodgkin's and non-Hodgkin's lymphoma, Burkitt's lymphoma, epithelial cell carcinomas including gastric cancer, as well as autoimmune diseases such as multiple sclerosis. The initial site for EBV replication is located in the oropharynx, after which EBV grows and multiplies in B lymphocytes and oral epithelial cells, then infects more B lymphocytes. These infected cells enter the bloodstream in large numbers, causing systemic infection. When the body's immune function is compromised, latent EBV will be activated, leading to recurrent infection.

Currently, there is no effective vaccine against EBV, and diseases caused by EBV infection also lack specific treatment approaches. Infectious mononucleosis is mostly treated with antiviral drugs such as acyclovir. Although these drugs can relieve symptoms to some extent, they cannot eliminate EBV in B lymphocytes and pharyngeal epithelium. Treatment for EBV-associated tumors is primarily chemotherapy and radiotherapy, but the efficacy is poor for patients with metastasis or recurrence.

Monoclonal antibodies can be massively produced, and by virtue of their high affinity and high specificity for antigen binding, they greatly reduce adverse reactions during clinical application. These antibody molecules can also be modified to increase their antiviral efficacy. Antibodies, by virtue of their specificity and flexibility of use, have become a very promising approach for the treatment of infectious diseases, but to date, no monoclonal antibody targeting the EBV envelope glycoprotein gB has been marketed. Therefore, the development of anti-EBV monoclonal antibodies will provide more effective prevention and treatment approaches for EBV infection-related diseases.

### SUMMARY

A first aspect of the present disclosure aims to provide a monoclonal antibody or an antigen-binding fragment thereof.

A second aspect of the present disclosure aims to provide a recombinant protein.

A third aspect of the present disclosure aims to provide a biological material related to the monoclonal antibody or the antigen-binding fragment thereof according to the first aspect or the recombinant protein according to the second aspect of the present disclosure.

A fourth aspect of the present disclosure aims to provide a method for preparing the monoclonal antibody or the antigen-binding fragment thereof according to the first aspect or the recombinant protein according to the second aspect of the present disclosure.

A fifth aspect of the present disclosure aims to provide a conjugate.

A sixth aspect of the present disclosure aims to provide use of the monoclonal antibody or the antigen-binding fragment thereof according to the first aspect, the recombinant protein according to the second aspect, the biological material according to the third aspect, and/or the conjugate according to the fifth aspect of the present disclosure in the preparation of a product.

A seventh aspect of the present disclosure aims to provide a drug.

An eighth aspect of the present disclosure aims to provide a vaccine.

To achieve the above objectives, the technical solutions used in the present disclosure are as follows.

The first aspect of the present disclosure provides a monoclonal antibody or an antigen-binding fragment thereof against EBV gB protein, the monoclonal antibody or the antigen-binding fragment thereof comprises a heavy chain variable region and a light chain variable region;
the heavy chain variable region comprises CDR-H1, CDR-H2, and CDR-H3;
the CDR-H1, CDR-H2, and CDR-H3 are CDR1, CDR2, CDR3 of an amino acid sequence shown in SEQ ID NO: 4;
the light chain variable region comprises CDR-L1, CDR-L2, and CDR-L3;
the CDR-L1, CDR-L2, and CDR-L3 are CDR1, CDR2, CDR3 of an amino acid sequence shown in SEQ ID NO: 17.

Preferably, amino acid sequences of the CDR-H1, CDR-H2, CDR-H3, CDR-L1, CDR-L3 are sequentially as shown in SEQ ID NO: 5, SEQ ID NO: 6, SEQ ID NO: 7, SEQ ID NO: 18, and SEQ ID NO: 19, an amino acid sequence of the CDR-L2 is DTS, and the CDRs are defined by an IMGT definition scheme.

Preferably, amino acid sequences of the CDR-H1, CDR-H2, CDR-H3, CDR-L1, CDR-L2, CDR-L3 are sequentially as shown in SEQ ID NO: 8, SEQ ID NO: 9, SEQ ID NO: 10, SEQ ID NO: 20, SEQ ID NO: 21, and SEQ ID NO: 19, and the CDRs are defined by a Kabat definition scheme.

Preferably, amino acid sequences of the CDR-H1, CDR-H2, CDR-H3, CDR-L1, CDR-L2, CDR-L3 are sequentially as shown in SEQ ID NO: 11, SEQ ID NO: 12, SEQ ID NO: 10, SEQ ID NO: 20, SEQ ID NO: 21, and SEQ ID NO: 19, and the CDRs are defined by a Chothia definition scheme.

Preferably, amino acid sequences of the CDR-H1, CDR-H2, CDR-H3, CDR-L1, CDR-L2, CDR-L3 are sequentially as shown in SEQ ID NO: 13, SEQ ID NO: 14, SEQ ID NO: 15, SEQ ID NO: 22, SEQ ID NO: 23, and SEQ ID NO: 24, and the CDRs are defined by a Contact definition scheme.

Preferably, an amino acid sequence of the heavy chain variable region comprises:
a1) SEQ ID NO: 4; or
a2) an amino acid sequence obtained by substituting and/or deleting and/or adding one or more amino acids in SEQ ID NO: 4, and having the same function as the protein shown in SEQ ID NO: 4; or
a3) an amino acid sequence having 99%, 98%, 97%, 96%, 95%, 94%, 93%, 92%, 91%, 90%, 89%, 88%, 87%, 86%, 85%, 84%, 83%, 82%, 81% or 80% homology with SEQ ID NO: 4, and having the same function as the protein shown in SEQ ID NO: 4.

Preferably, an amino acid sequence of the light chain variable region comprises:
b1) SEQ ID NO: 17; or
b2) an amino acid sequence obtained by substituting and/or deleting and/or adding one or more amino acids in SEQ ID NO: 17, and having the same function as the protein shown in SEQ ID NO: 17; or
b3) an amino acid sequence having 99%, 98%, 97%, 96%, 95%, 94%, 93%, 92%, 91%, 90%, 89%, 88%, 87%, 86%, 85%, 84%, 83%, 82%, 81% or 80% homology with SEQ ID NO: 17, and having the same function as the protein shown in SEQ ID NO: 17.

Preferably, the monoclonal antibody or the antigen-binding fragment thereof comprises at least one of a full-length antibody, Fab, Fab', F(ab')2, Fv, scFv, a bispecific antibody, or a multispecific antibody.

Preferably, the heavy chain further comprises a heavy chain constant region; and/or, the light chain further comprises a light chain constant region.

Preferably, an amino acid sequence of the heavy chain constant region comprises:
c1) an amino acid sequence consisting of amino acids at positions 138 to 467 in SEQ ID NO: 3; or
c2) an amino acid sequence obtained by substituting and/or deleting and/or adding one or more amino acids in the amino acid sequence of c1), and having the same function as the protein of the amino acid sequence of c1); or
c3) an amino acid sequence having 99%, 98%, 97%, 96%, 95%, 94% or 93% homology with the amino acid sequence of c1), and having the same function as the protein of the amino acid sequence of c1).

Preferably, an amino acid sequence of the light chain constant region comprises:
d1) an amino acid sequence consisting of amino acids at positions 127 to 232 in SEQ ID NO: 16; or
d2) an amino acid sequence obtained by substituting and/or deleting and/or adding one or more amino acids in the amino acid sequence of d1), and having the same function as the protein of the amino acid sequence of d1); or
d3) an amino acid sequence having 99%, 98%, 97%, 96%, 95%, 94% or 93% homology with the amino acid sequence of d1), and having the same function as the protein of the amino acid sequence of d1).

Preferably, the heavy chain further comprises a heavy chain signal peptide; and/or, the light chain further comprises a light chain signal peptide.

Preferably, an amino acid sequence of the heavy chain signal peptide comprises:
e1) an amino acid sequence consisting of amino acids at positions 1 to 19 of SEQ ID NO: 3; or
e2) an amino acid sequence obtained by substituting and/or deleting and/or adding one or more amino acids in the amino acid sequence of e1), and having the same function as the protein of the amino acid sequence of e1); or
e3) an amino acid sequence having 99%, 98%, 97%, 96%, 95%, 94% or 93% homology with the amino acid sequence of e1), and having the same function as the protein of the amino acid sequence of e1).

Preferably, an amino acid sequence of the light chain signal peptide comprises:
f1) an amino acid sequence consisting of amino acids at positions 1 to 19 of SEQ ID NO: 16; or
f2) an amino acid sequence obtained by substituting and/or deleting and/or adding one or more amino acids in the amino acid sequence of f1), and having the same function as the protein of the amino acid sequence of f1); or
f3) an amino acid sequence having 99%, 98%, 97%, 96%, 95%, 94% or 93% homology with the amino acid sequence of f1), and having the same function as the protein of the amino acid sequence of f1).

Preferably, an amino acid sequence of the EBV gB protein comprises:
g1) an amino acid sequence consisting of amino acids at positions 25 to 685 of SEQ ID NO: 1; or
g2) an amino acid sequence obtained by substituting and/or deleting and/or adding one or more amino acids in the amino acid sequence of g1), and having the same function as the protein of the amino acid sequence of g1); or
g3) an amino acid sequence having 99%, 98%, 97%, 96%, 95%, 94% or 93% homology with the amino acid sequence of g1), and having the same function as the protein of the amino acid sequence of g1).

The second aspect of the present disclosure provides a recombinant protein, comprising the monoclonal antibody or the antigen-binding fragment thereof according to the first aspect of the present disclosure; and optionally a tag sequence that assists expression and/or purification.

Preferably, the tag sequence is at least one selected from the group consisting of a histidine (His) tag, a GGGS sequence (SEQ ID NO: 25), and a FLAG tag; more preferably a His tag; even more preferably a 6×His tag.

The third aspect of the present disclosure provides a biological material related to the monoclonal antibody or the antigen-binding fragment thereof according to the first aspect of the present disclosure, or the recombinant protein according to the second aspect of the present disclosure, the biological material comprises at least one of h1) to h16):
h1) a nucleic acid molecule encoding the monoclonal antibody or the antigen-binding fragment thereof according to the first aspect, or the recombinant protein according to the second aspect of the present disclosure;
h2) an expression cassette comprising the nucleic acid molecule of h1);
h3) a vector comprising the nucleic acid molecule of h1);
h4) a vector comprising the expression cassette of h2);
h5) a transgenic cell line comprising the nucleic acid molecule of h1);
h6) a transgenic cell line comprising the expression cassette of h2);
h7) a transgenic cell line comprising the vector of h3);
h8) a transgenic cell line comprising the vector of h4);
h9) a microorganism comprising the nucleic acid molecule of h1);
h10) a microorganism comprising the expression cassette of h2);
h11) a microorganism comprising the vector of h3);
h12) a microorganism comprising the vector of h4);
h13) a virus comprising the nucleic acid molecule of h1);
h14) a virus comprising the expression cassette of h2);
h15) a virus comprising the vector of h3);
h16) a virus comprising the vector of h4).

Preferably, the transgenic cell line does not comprise a propagation material.

Preferably, the nucleic acid molecule encoding the monoclonal antibody or the antigen-binding fragment thereof according to the first aspect of the present disclosure comprises:
a nucleic acid molecule encoding the heavy chain of the monoclonal antibody or the antigen-binding fragment thereof according to the first aspect of the present disclosure; and
a nucleic acid molecule encoding the light chain of the monoclonal antibody or the antigen-binding fragment thereof according to the first aspect of the present disclosure.

The fourth aspect of the present disclosure provides a method for preparing the monoclonal antibody or the antigen-binding fragment thereof according to the first aspect or the recombinant protein according to the second aspect of the present disclosure, the method comprises the following steps: expressing and then purifying the biological material according to the third aspect of the present disclosure to obtain the monoclonal antibody or the antigen-binding fragment thereof or the recombinant protein.

The fifth aspect of the present disclosure provides a conjugate, comprising at least one of the monoclonal antibody or the antigen-binding fragment thereof according to the first aspect of the present disclosure, or the recombinant protein according to the second aspect of the present disclosure; and a conjugated moiety, the conjugated moiety comprises at least one of a detectable label, a drug, a toxin, a cytokine, a radionuclide, or an enzyme.

Preferably, the detectable label is selected from the group consisting of a radioisotope, a fluorescent substance, a chemiluminescent substance, a colored substance, and any combination thereof.

Preferably, the conjugate is selected from the group consisting of a fluorescent substance, a chemiluminescent label, a colored substance, a radioisotope, a magnetic resonance imaging (MRI) or computed tomography (CT) contrast agent, an enzyme capable of producing a detectable product, a radionuclide, a biotoxin, a cytokine (e.g., IL-2, etc.), an antibody, an antibody Fc fragment, an antibody scFv fragment, gold nanoparticles/nanorods, viral particles, liposomes, magnetic nanoparticles, a prodrug-activating enzyme, a chemotherapeutic agent (e.g., cisplatin), and nanoparticles of any form.

The sixth aspect of the present disclosure provides use of the monoclonal antibody or the antigen-binding fragment thereof according to the first aspect, the recombinant protein according to the second aspect, the biological material according to the third aspect, and/or the conjugate according to the fifth aspect of the present disclosure in the preparation of a product;
the product comprises at least one of a drug, a reagent, a detection plate, a kit, or a detection chip.

Preferably, the drug has at least one of the following functions i1) to i2):
i1) preventing EBV infection;
i2) treating and/or preventing a disease caused by EBV infection.

Preferably, the reagent, detection plate, detection chip or kit has at least one of the following functions j1) to j3):
j1) detecting a presence or level of gB protein in a sample;
j2) detecting EBV;
j3) diagnosing a disease caused by EBV infection.

Preferably, the disease comprises at least one of nasopharyngeal carcinoma, gastric cancer, Hodgkin's lymphoma, Burkitt's lymphoma, NK/T cell lymphoma, lymphoproliferative disease, or infectious mononucleosis.

Preferably, the drug comprises a pharmaceutically acceptable excipient.

Preferably, the drug comprises a vaccine.

Preferably, the vaccine comprises a pharmaceutically acceptable adjuvant.

The seventh aspect of the present disclosure provides a product, and the product comprises at least one of k1) to k3):
k1) the monoclonal antibody or the antigen-binding fragment thereof according to the first aspect of the present disclosure;
k2) the recombinant protein according to the second aspect of the present disclosure;
k3) the conjugate according to the fifth aspect of the present disclosure;
the product comprises at least one of a reagent, a detection plate, a kit, or a detection chip.

Preferably, the product has at least one of the following functions j1) to j3):
j1) detecting a presence or level of gB protein in a sample;
j2) detecting EBV;
j3) diagnosing a disease caused by EBV infection.

Preferably, the disease comprises at least one of nasopharyngeal carcinoma, gastric cancer, Hodgkin's lymphoma, Burkitt's lymphoma, NK/T cell lymphoma, lymphoproliferative disease, or infectious mononucleosis.

The eighth aspect of the present disclosure provides a drug, and the drug comprises at least one of l1) to l4):
l1) the monoclonal antibody or the antigen-binding fragment thereof according to the first aspect of the present disclosure;
l2) the recombinant protein according to the second aspect of the present disclosure;
l3) the biological material according to the third aspect of the present disclosure;
l4) the conjugate according to the fifth aspect of the present disclosure.

Preferably, the drug further comprises a pharmaceutically acceptable carrier.

Preferably, the drug has at least one of the following functions i1) to i2):
i1) preventing EBV infection;
i2) treating and/or preventing a disease caused by EBV infection.

Preferably, the disease comprises at least one of nasopharyngeal carcinoma, gastric cancer, Hodgkin's lymphoma, Burkitt's lymphoma, NK/T cell lymphoma, lymphoproliferative disease, or infectious mononucleosis.

Preferably, the drug comprises a vaccine.

Preferably, the vaccine comprises at least one of l1) to l4) and an adjuvant:
l1) the monoclonal antibody or the antigen-binding fragment thereof according to the first aspect of the present disclosure;
l2) the recombinant protein according to the second aspect of the present disclosure;
l3) the biological material according to the third aspect of the present disclosure;
l4) the conjugate according to the fifth aspect of the present disclosure.

The beneficial effects of the present disclosure are as follows.

The present disclosure provides a monoclonal antibody or an antigen-binding fragment thereof against EBV gB. This monoclonal antibody or antigen-binding fragment thereof has high affinity for EBV gB protein, and can significantly inhibit EBV infection of epithelial cells and B cells. It can be used for detecting a presence or level of gB protein in a sample, detecting EBV, diagnosing a disease caused by EBV infection, and preventing EBV infection and/or treating and/or preventing a disease caused by EBV infection.

### BRIEF DESCRIPTION OF DRAWINGS

FIG. 1 is a graph showing the affinity detection result of monoclonal antibody Fab5 with gB protein.
FIG. 2 is a graph showing the result of monoclonal antibody Fab5 blocking EBV infection on epithelial cells.
FIG. 3 is a graph showing the result of monoclonal antibody Fab5 blocking EBV infection on B cells.
FIG. 4 is a graph showing the result of the cryo-electron microscopy structure of monoclonal antibody Fab5 in complex with gB protein.

### DETAILED DESCRIPTION

The concept and resulting technical effects of the present disclosure will be clearly and completely described below in conjunction with the examples to fully understand the objectives, features, and effects of the present disclosure. It is apparent that the described examples are only a part of the examples of the present disclosure, rather than all examples. Based on the examples of the present disclosure, other examples obtained by those skilled in the art without creative efforts shall fall within the protection scope of the present disclosure.

In the following examples, experimental methods without specified conditions are generally performed under conventional conditions or under conditions recommended by the manufacturer. The materials and reagents used in these examples, unless otherwise specified, are all commercially available reagents and materials.

### Example 1: Preparation of anti-EBV gB protein (EBV gB), animal immunization, and antibody screening

### 1.1 Expression and purification of EBV gB antigen protein

### 1.1.1 Experimental materials

(1) Expression vector: eukaryotic expression vector pcDNA3.1(+) (Thermo Fisher).
(2) Expression system: eukaryotic expression system: HEK293F cells (ATCC).
(3) Reagents and consumables: plasmid midiprep kit (MN), cell transfection reagent PEI (Polyscience), 293F medium (Union), histidine-tagged protein purification agarose beads (Roche), etc., and other conventional reagents and consumables were all purchased from commercial sources.
(4) Gene: the gB gene of Epstein-Barr virus (M81 strain) was optimized and synthesized by Nanjing GenScript Biotechnology Co., Ltd., China and inserted into the pcDNA3.1 vector to construct a recombinant plasmid, hereinafter referred to as pcDNA3.1-gB.

The recombinant protein of the gB ectodomain has a full length of 661 amino acid residues (* not included), wherein the signal peptide is marked in bold. The gB purified protein has the specific sequence as follows:

MPMGSLQPLATLYLLGMLVASCLGQTPEQPAPPATTVQPTATRQQTSFPFRVCELSSH
GDLFRFSSDIQCPSFGTRENHTEGLLMVFKDNIIPYSFKVRSYTKIVTNILIYNGHRADSVTN
RHEEKFSVESYETDQMDTIYQCYNAVKMTKDGLTRVYVDRDGVNITVNLKPTGGLANGV
RRYASQTELYDAPGRVEATYRTRTTVNCLITDMMAKSNSPFDFFVTTTGQTVEMSPFYDGK
NTETFHERADSFHVRTNYKIVDYDNRGTNPQGERRAFLDKGTYTLSWKLENRTAYCPLQH
WQTFDSTIATETGKSIHFVTDEGTSSFVTNTTVGIELPDAFKCIEEQVNKTMHEKYEAVQDR
YTKGQEAITYFITSGGLLLAWLPLTPRSLATVKNLTELTTPTSSPPSSPSPPAPPAARGSTSAAV
LRRRRRNAGNATTPVPPAAPGKSLGTLNNPATVQIQFAYDSLRRQINRMLGDLARAWCLEQ
KRQNMVLRELTKINPTTVMSSIYGKAVAAKRLGDVISVSQCVPVNQATVTLRKSMRVPGSE
TMCYSRPLVSFSFINDTKTYEGQLGTDNEIFLTKKMTEVCQATSQYYFQSGNEIHVYNDYH
HFKTIELDGIATLQTFISLNTSLIENIDFASLELYSRDEQRASNVFDLEGIFREYNFQAQNIAGL
RKDLDNAVSHHHHHH* (SEQ ID NO: 1).

The DNA expression sequence has 2071 base pairs in total, wherein the signal peptide is marked in bold, with the specific sequence as follows:

ATGCCAATGGGCTCTCTGCAGCCCCTGGCCACACTGTACCTGCTGGGAATGCT
GGTGGCAAGCTGCCTGGGACAGACCCCAGAGCAGCCTGCACCACCTGCCACCACAG
TGCAGCCTACAGCCACCAGACAGCAGACCAGCTTCCCCTTCCGGGTGTGCGAGCTGAG
CTCCCACGGCGACCTGTTCAGATTTTCTAGCGATATCCAGTGTCCCTCCTTCGGCACAAG

GGAGAACCACACCGAGGGCCTGCTGATGGTGTTCAAGGACAATATCATCCCTTACTCCT
TTAAGGTGAGATCTTATACAAAGATCGTGACCAACATCCTGATCTACAATGGCCACAGAG
CCGATTCTGTGACCAACAGGCACGAGGAGAAGTTTAGCGTGGAGTCCTATGAGACAGA
CCAGATGGATACCATCTACCAGTGCTATAATGCCGTGAAGATGACAAAGGACGGCCTGA
CCAGAGTGTACGTGGACAGGGATGGCGTGAACATCACAGTGAATCTGAAGCCTACCGG
AGGACTGGCAAACGGCGTGCGGAGATACGCCAGCCAGACCGAGCTGTATGATGCACCA
GGAAGGGTGGAGGCAACATACAGGACCAGGACCACAGTGAACTGTCTGATCACAGACA
TGATGGCCAAGAGCAATTCCCCATTCGATTTCTTTGTGACCACAACCGGCCAGACCGTG
GAGATGAGCCCCTTTTATGACGGCAAGAACACAGAGACCTTCCACGAGCGCGCCGATTC
CTTTCACGTGCGGACAAATTACAAGATCGTGGACTATGATAACAGAGGAACCAATCCAC
AGGGAGAGAGGAGGGCCTTCCTGGACAAGGGCACATACACCCTGTCCTGGAAGCTGGA
GAACAGGACAGCCTATTGCCCCCTGCAGCACTGGCAGACATTTGACTCCACCATCGCCA
CAGAGACCGGCAAGTCTATCCACTTCGTGACAGATGAGGGCACCTCCTCTTTTGTGACC
AACACAACCGTGGGCATCGAGCTGCCCGACGCCTTCAAGTGTATCGAGGAGCAAGTGA
ATAAGACAATGCACGAGAAGTACGAGGCCGTGCAGGATCGCTATACCAAGGGCCAGGA
AGCCATCACATACTTTATCACCAGCGGAGGACTGCTGCTGGCATGGCTGCCACTGACAC
CACGGTCCCTGGCCACAGTGAAGAATCTGACCGAGCTGACAACCCCAACCAGCTCCCC
ACCATCTAGCCCAAGCCCTCCAGCACCACCTGCAGCACGGGGCTCTACCAGCGCCGCCG
TGCTGCGGAGAAGGCGCCGGAACGCAGGAAATGCAACAACCCCTGTGCCACCAGCAG
CACCTGGCAAGTCTCTGGGCACACTGAACAATCCCGCCACCGTGCAGATCCAGTTCGCC
TACGACAGCCTGAGAAGGCAGATCAACAGAATGCTGGGCGATCTGGCAAGGGCATGGT
GCCTGGAGCAGAAGCGCCAGAACATGGTGCTGCGGGAGCTGACCAAGATCAATCCTAC
AACCGTGATGTCCTCTATCTATGGCAAGGCAGTGGCAGCAAAGAGACTGGGCGACGTG
ATCTCCGTGTCTCAGTGCGTGCCAGTGAATCAGGCCACAGTGACCCTGAGAAAGAGCAT
GAGGGTGCCCGGCTCCGAGACCATGTGCTACTCTAGGCCTCTGGTGAGCTTCTCCTTTAT
CAACGACACAAAGACCTATGAGGGCCAGCTGGGCACAGATAATGAGATCTTCCTGACA
AAGAAGATGACCGAGGTGTGCCAGGCCACCAGCCAGTACTATTTCCAGTCCGGCAACG
AGATCCACGTGTACAATGACTATCACCACTTTAAGACAATCGAGCTGGATGGCATCGCCA
CACTGCAGACCTTCATCTCTCTGAACACCAGCCTGATCGAGAATATCGACTTTGCCAGCC
TGGAGCTGTACTCCAGGGACGAGCAGAGGGCATCCAACGTGTTCGATCTGGAGGGCAT
CTTCCGCGAGTATAACTTTCAGGCCCAGAATATCGCCGGCCTGCGGAAGGACCTGGATA
ATGCCGTGTCTCACCACCACCACCACCAC (SEQ ID NO: 2).

### 1.1.2 Protein expression

Step 1: Transformation and picking of single clones
(1) The synthesized plasmid was added to thawed *Escherichia coli* DH5α, and was left to stand on ice for 5 minutes.
(2) A resulting bacterial liquid was placed in a shaking metal bath and was heat-shocked at 42 °C, 120 rpm for 60-90 seconds.
(3) A resulting bacterial liquid was left to stand on ice for cooling for 5 minutes.
(4) The bacterial liquid was spread onto an ampicillin-resistant Terrific Broth (TB) medium plate using a sterile spreading rod, and was cultured overnight.
(5) On the next day, a single colony on the plate surface was picked using a sterile pipette tip and was transferred into 500 mL of medium, 0.1 mg/mL ampicillin was added to the medium, and a resulting mixture was cultured at 37 °C, 220 rpm for 12-16 hours.

Step 2: Plasmid extraction
(1) The cultured bacterial liquid was centrifuged at 4000 rpm, 4 °C, the supernatant was discarded, and a resulting bacterial pellet was collected. Plasmid extraction was then performed according to the instructions of the MN midiprep kit. A brief procedure for plasmid extraction was as follows.
(2) The pellet was resuspended using the RES resuspension buffer (buffer, containing RNase A) from the MN midiprep kit.
(3) LYS lysis buffer was added, and lysis was carried out for 5 minutes.
(4) NEU neutralization buffer was added for neutralization, at which point a precipitate was produced and the solution became clear yellow.
(5) Hollow fiber column was equilibrated using EQU equilibrium buffer, taking care that the solution flowed from the top inlet of the fiber column to completely wet the entire fiber sleeve.
(6) The neutralized solution together with the precipitate was transferred into the equilibrated hollow fiber column.
(7) EQU equilibrium buffer was added to re-equilibrate the hollow fiber column until the solution beneath the column became clear, colorless, and transparent.
(8) The intermediate fiber sleeve was discarded, and the outer adsorption column was left.
(9) Wash buffer was added to wash the adsorption membrane below the adsorption column.
(10) A centrifuge tube was placed under the adsorption column to collect the flow-through, then an appropriate amount of Elu elution buffer was added to elute the plasmid, and finally isopropanol was added to precipitate the plasmid.
(11) The precipitated plasmid solution was centrifuged at 4000 rpm for 30 minutes, the supernatant was discarded, a resulting precipitate was resuspended in 70% ethanol and centrifugated again.
(12) For the centrifuged mixture, the supernatant was discarded in a clean bench, and the plasmid was resuspended in an appropriate amount of sterilized double-distilled water. The resuspended plasmid was stored at -20 °C.

Step 3: Plasmid transfection of 293F cells
(1) 293F cells were cultured with shaking in medium at 37 °C, 120 rpm, 5% CO₂, and the density was maintained at 0.5-5×10⁶ cells/mL (0.5-5X). When the density exceeded this range, subculturing or distribution for transfection was performed in a timely manner.
(2) The plasmid was diluted with 293 medium. Generally, the transfection amount was calculated by transfecting 1 mg of plasmid per 1 L of 1X cells.
(3) Dissolved PEI was diluted with 293 medium. Generally, the transfection system was prepared by adding 5 mg of PEI per 1 mg of plasmid.
(4) The plasmid diluted with 293 medium and the PEI were mixed and allowed to stand for 10-15 minutes.
(5) The density of 293F cells was diluted to 1X. The 293F cells were added with the transfection mixture system and continuously cultured with shaking at 37 °C, 120 rpm, 5% CO₂ for 5-7 days.

Step 4: Purification of secreted protein from eukaryotic culture supernatant
(1) The cultured 293F cells were centrifuged at 8000 rpm, 4 °C for 1 hour, and the supernatant was collected. Since the synthesized plasmid contained a signal peptide sequence, the protein of interest was secreted into the supernatant; therefore, the supernatant was used for protein purification.
(2) The cell supernatant was filtered twice through a 0.22 µm filter membrane to remove impurities.
(3) A gravity purification column was added with 1 mL of His-tagged protein purification agarose beads, and double-distilled water filtered through a 0.22 µm filter membrane (all liquids added to the purification column during the purification step were required to be filtered through a 0.22 µm filter membrane) was added to wash the beads. Then, the purification column was equilibrated with phosphate-buffered saline (PBS).
(4) The supernatant was transferred to the purification column by a siphon method, so that the supernatant completely passed through the purification column. If multiple recoveries were needed, the supernatant could be recovered at this step and purified again.
(5) The purification column was equilibrated with three column volumes of PBS.
(6) The purification column was eluted with two column volumes of PBS containing 300 mM imidazole, and a resulting eluate was collected.
(7) Samples of the supernatant, equilibration solution, and eluate were respectively retained, and protein expression was verified by sodium dodecyl sulfate-polyacrylamide gel electrophoresis (SDS-PAGE).
(8) The eluate was concentrated using an ultrafiltration tube with an appropriate molecular weight cutoff so that the concentrated volume was less than 1 mL. The concentrated eluate was centrifuged at 12,000g, 4 °C for 5 minutes to remove precipitate.
(9) The concentrated eluate was subjected to size exclusion chromatography using an AKTA purifier equipped with a Superdex 200 Increase 10/300 GL size exclusion column. Protein sample at the peak position of the size exclusion chromatography was collected, and was verified by SDS-PAGE together with the previous samples.

### 1.2 Immunization experiment for EBV gB mouse

### 1.2.1 Vaccine preparation

(1) The gB protein was thawed. A dose of gB was 5 µg per mouse. An antigen solution was prepared using sterile PBS as the solvent.
(2) 100 µg of the antigen solution was mixed with 100 µL of Complete Freund's Adjuvant (CFA). The mixture was emulsified to form a dose of 200 µL per mouse, and was placed on an inversion shaker at 4 °C to mix overnight.

### 1.2.2 Mouse immunization and spleen collection

(1) Mice were fed with conventional feed and conventional drinking water. Immunization was performed at 6 weeks of age, and the strain used was Balb/C.
(2) The mouse was fixed. The vaccine (manually re-shaken) was aspirated using a 1 mL syringe and then administered via abdominal subcutaneous injection.
(3) The mouse was observed for 3-5 minutes, and was returned to its housing cage after normal activity resumed.
(4) The vaccine immunization schedule was performed as follows: week 0, week 3, week 6, and week 8. The spleen was collected at week 10.
(5) When collecting the mouse spleen, according to animal welfare and standard animal experimental procedures, the mouse was sacrificed by cervical dislocation. Its blood was collected, and its spleen was obtained by dissection.

### 1.3 Isolation and cloning of EBV gB mouse B cell gB-specific cells

### 1.3.1 Spleen lymphocyte isolation steps:

(1) At week 10 after immunization, the mouse was sacrificed by cervical dislocation. Its peripheral lymph nodes and spleen were isolated and placed into an EP tube.
(2) Appropriate amounts of DNase and type IV collagenase were added to 1640 medium to prepare a tissue digestion solution. An appropriate amount of digestion solution was added according to the size of the lymph nodes and spleen.
(3) The tissue was placed on a 70 µm cell strainer. A sterile syringe plunger was used to crush the tissue until it was almost fragmented. Then the cell strainer was rinsed with an appropriate amount of fresh medium to collect all remaining cells into a 50 mL centrifuge tube below.
(4) Red blood cell lysis buffer was added, and a resulting mixture was incubated at room temperature for 10 minutes.
(5) Centrifugation was performed at 2000g, 18 °C for 5 minutes, and the supernatant was discarded.
(6) The cells were washed twice with fresh medium.

### 1.3.2 Staining and sorting of B lymphocyte antigen

(1) The cells were washed with PBS and adjusted to 1×10⁸ cells per flow tube. Centrifugation was performed at 3000g, 18 °C for 5 minutes, and the supernatant was discarded.
(2) 5 µL of Fc receptor blocking antibody solution was added to 100 µL of PBS. The cells were resuspended and incubated on ice for 30 minutes.
(3) Antibody diluent for flow cytometry was prepared with PBS according to the cell groupings. When the antibody was used for the first time, different dilution gradients were set. Tests were performed at ratios of antibody: PBS of 1:20, 1:50, 1:100, 1:200, and 1:500, and the optimal gradient was selected. 100 µL of the diluted antibody for flow cytometry was mixed with dye for cell viability. The cells were resuspended for staining and were incubated at 4 °C in the dark for 30 minutes. Mouse IgG and B220 antibodies were used as B cell isolation reagents, and gB was used as the gB-specific B cell isolation reagent.
(4) The cells were washed with PBS. Centrifugation was performed at 3000g, 4 °C for 5 minutes. The washing was repeated 1-2 times, and the supernatant was discarded.
(5) The cells were resuspended in PBS into flow cytometry tubes, and were sorted by flow cytometry using a BD Rhapsody single-cell sorter.
(6) The 0.2% of cells with the strongest gB positive signal among the IgG⁺B220⁺ cells were sorted into a 96-well plate.

### 1.3.3 Cloning and amplification of B cell sequence

(1) PCR was performed on B cells in the 96-well plate using mouse IgG heavy chain and light chain universal cloning primers to obtain the heavy chain and light chain sequences of the corresponding single B cell.
(2) The upstream of heavy chain variable region of the antibody was connected to a cytomegalovirus (CMV) fragment, and the downstream was connected to the constant region of mouse IgG1, so that a recombinant fragment capable of expressing the complete heavy chain was constructed; whereas the upstream of the light chain variable region of the antibody was connected to a CMV fragment, and the downstream was connected to the constant region of light chain κ/λ, so that a recombinant fragment capable of expressing the complete light chain was constructed. Plasmids containing the full-length sequences of the above antibody heavy chain and light chain were co-transfected into 293F cells, whereby the expression of the antibody was achieved. The antibody was purified using protein A beads. A total of 8 antibodies (Fab1-Fab8) were obtained by this method.
(3) gB affinity determination of the obtained antibodies was performed using enzyme-linked immunosorbent assay (ELISA). The Fab5 antibody with the highest affinity was screened out and was used for further validation.

### Example 2: Preparation of anti-EBV gB protein (EBV gB) monoclonal antibody (mAb)

The upstream of heavy chain variable region of the antibody was connected to a CMV fragment, and the downstream was connected to the constant region of human IgG1, so that a recombinant fragment capable of expressing the complete heavy chain was constructed; whereas the upstream of light chain variable region of the antibody was connected to a CMV fragment, and the downstream was connected to the constant region of light chain κ/λ, so that a recombinant fragment capable of expressing the complete light chain was constructed. Plasmids containing the full-length sequences of the above antibody heavy chain and light chain were co-transfected into 293F cells, whereby the expression of the antibody was achieved. The antibody was purified using protein A beads.

The full-length heavy chain of Fab5 has a total of 467 amino acid residues (*is not included), with the specific sequence as follows: 
MGWSCIILFLVATATGVHSQVQLQQPGAELVRPGASVKLSCKASGFTFTSYWINWVK
QRPGQGLEWIGNIFPSDSYTNYNQKFKDKATLTVDKSSSTAYMLLSSPTSEDSAVYYCTRG
GYRYDSDYWGQGTTLTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGK* (SEQ ID NO: 3);
 wherein the underlined part of the sequence is the amino acid sequence of the heavy chain variable region (SEQ ID NO: 4). The underlined and italicized parts are sequentially the amino acid sequences of the three complementarity regions CDR-H1 (SEQ ID NO: 5), CDR-H2 (SEQ ID NO: 6), and CDR-H3 (SEQ ID NO: 7) in the heavy chain variable region (IMGT definition scheme). The bold part is the signal peptide sequence. Amino acids at positions 138 to 467 represent the heavy chain constant region. * indicates a stop codon.

The sequences of CDR-H1, CDR-H2, CDR-H3 in this heavy chain variable region using other CDR definition schemes are shown in Table 11.

**Table 11: Sequences of CDR-H1, CDR-H2, CDR-H3 in the heavy chain variable region using other CDR definition schemes**

| Other CDR definition scheme | CDR-H1 | CDR-H2 | CDR-H3 |
|---|---|---|---|
| Kabat | SYWIN (SEQ ID NO:8) | NIFPSDSYTNYNQKFK D (SEQ ID NO: 9) | GGYRYDSDY (SEQ ID NO: 10) |
| Chothia | GFTFTSY (SEQ ID NO:11) | FPSDSY (SEQ ID NO:12) | GGYRYDSDY (SEQ ID NO: 10) |
| Contact | TSYWIN (SEQ ID NO:13) | WIGNIFPSDSYTN (SEQ ID NO: 14) | TRGGYRYDSD (SEQ ID NO: 15) |

The full-length light chain of Fab5 has a total of 232 amino acid residues (* is not included), with the specific sequence as follows: 
MGWSCIILFLVATATGSWAQIVLTQSPAIMSASPGEKVTMTCSASSSVTYMYWYQQKP
GSSPRLLIYDTSNLASGVPVRFSGSGSGTSYSLTISRMEAEDAATYYCQQWSSYPYTFGGGT
KLEIKRTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC* (SEQ ID NO: 16);
 wherein the underlined part of the sequence is the amino acid sequence of the light chain variable region (SEQ ID NO: 17). The underlined and italicized parts are sequentially the amino acid sequences of the three complementarity determining regions CDR-L1 (SEQ ID NO: 18), CDR-L2 (DTS), and CDR-L3 (SEQ ID NO: 19) in the light chain variable region (IMGT definition scheme). The bold part is the signal peptide sequence. Amino acids at positions 127 to 232 represent the light chain constant region. * indicates a stop codon.

The sequences of CDR-L1, CDR-L2, CDR-L3 in this light chain variable region using other CDR definition schemes are shown in Table 12.

**Table 12: Sequences of CDR-L1, CDR-L2, CDR-L3 in the light chain variable region using other CDR definition schemes**

| Other CDR definition scheme | CDR-L1 | CDR-L2 | CDR-L3 |
|---|---|---|---|
| Kabat | SASSSVTYMY (SEQ ID NO: 20) | DTSNLAS (SEQ ID NO: 21) | QQWSSYPYT (SEQ ID NO: 19) |
| Chothia | SASSSVTYMY (SEQ ID NO: 20) | DTSNLAS (SEQ ID NO: 21) | QQWSSYPYT (SEQ ID NO: 19) |
| Contact | TYMYWY (SEQ ID NO: 22) | LLIYDTSNLA (SEQ ID NO: 23) | QQWSSYPY (SEQ ID NO: 24) |

### Example 3: Affinity determination of Fab5

The affinity of antibody Fab5 was determined using Bio-Layer Interferometry (BLI).

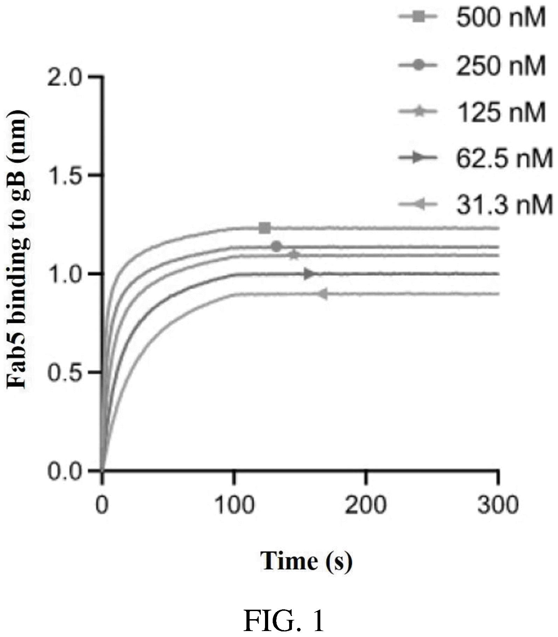

BLI could be performed according to conventional methods in the art. In this example, the specific operations were as follows: A biosensor (Sartorius Octet^{®} SA probe, Sartorius, Germany) was immersed in a buffer (a mixture of KB buffer, 0.1 wt% BSA, and 0.02 v/v% Tween 20) for equilibration. Then, the biosensor was removed and immersed in a solution containing 5 µg/mL gB-Biotin (biotin-labeled gB protein). The gB antigen in the solution was bound to the surface of the streptavidin (SA) biosensor probe, which caused an increase in the thickness of surface film. Then the biosensor with the immobilized gB antigen at a known concentration was immersed in the buffer, and the baseline signal was recorded. By immersing the biosensor with the immobilized antigen at a known concentration into a sample solution containing 31.3-500 nM Fab5 antibody for about 120 seconds, the film thickness was increased due to the specific antigen-antibody binding. The biosensor that had bound the Fab5 antibody was immersed in the buffer for dissociation for about 180 seconds. The test antibody (Fab5 antibody) was dissociated from the biosensor surface, causing the film thickness to decrease. Through real-time monitoring of the thickness of biosensor's biofilm during the experiment, the kinetic constant of the test sample (Fab5 antibody) was obtained. The results are shown in FIG. 1: the KD (M) of the Fab5 antibody is less than 10⁻¹² M (<10⁻¹² M), indicating that the Fab5 antibody has a very high affinity for the gB antigen.

### Example 4: Neutralization activity assay of Fab5 antibody

(1) Preparation of EBV virus:
   1) CNE2 cells infected with EBV-GFP (the virus was disclosed in the literature: "An Antibody Targeting the Fusion Machinery Neutralizes Dual-Tropic Infection and Defines a Site of Vulnerability on Epstein-Barr Virus") were cultured in RPMI1640 medium supplemented with 5 v/v% FBS in a 37 °C incubator (5 v/v% CO₂). When the cell density reached 90% (10 cm dish), phorbol-12-myristate-13-acetate (TPA or PMA) at a final concentration of 20 ng/mL and sodium butyrate (NaB) at a final concentration of 2.5 mM were added for induction. The medium was changed after 12 hours.
   2) At 48-72 hours after the medium change, the culture supernatant was collected, and the virus was isolated and purified. The supernatant was directly aspirated, centrifuged, and filtered through a 0.45 µm small filter. The filtrate was concentrated, resuspended in serum-free RPMI1640, and then immediately used for infection or was stored at -80 °C.
(2) Neutralization activity assay of the Fab5 monoclonal antibody on epithelial cells
   1) 1×10⁶ 293T epithelial cells were seeded per well in a 96-well plate, and 100 µL of DMEM medium containing 10% FBS was added to each well.
   2) On the next day, the Fab5 monoclonal antibody from the above example was adjusted to a concentration of 2 mg/mL. In a new 96-well plate, 60 µL of DMEM medium was added to each well. 120 µL of 12.5 µg/mL Fab5 antibody diluted with DMEM was added to the first well (the first well did not contain RPMI1640 medium).
   3) After 2-fold serial dilution (the serial dilution procedure was as follows: 60 µL was aspirated from the first well and added to the second well, and so on; 60 µL was aspirated from the last well and discarded; a final volume per well was 60 µL), 60 µL of virus diluent (the virus was diluted with DMEM medium to a titer of approximately 4×10⁶/mL) was added to each well, incubated at 37 °C for 2 hours, and a resulting mixture was added to the 293T cells that had been plated the previous day. The cells were placed in a 37 °C incubator and cultured for 48 hours for the assay.
   4) The 293T cells were digested with trypsin to prepare a cell suspension. The infection rate was detected by flow cytometry. By measuring the reduction ratio in the number of GFP-positive cells in the antibody-treated group compared to the infection control group (to which an equal volume of DMEM was added), the inhibition rate (neutralization efficiency, %) of the antibody in the 293T epithelial cell infection model was calculated. By using Prism software for plotting and calculation, the IC₅₀ of the Fab5 monoclonal antibody was obtained.

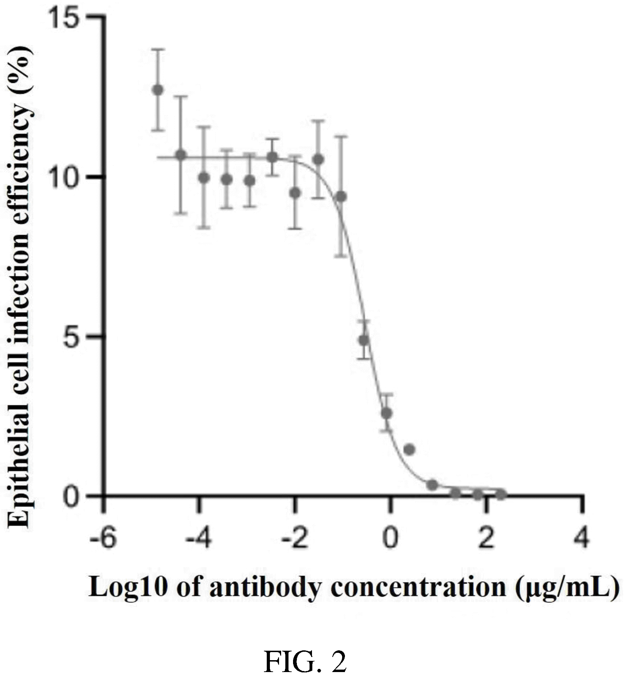

The results are shown in FIG. 2: the IC₅₀ of the monoclonal antibody Fab5 in the epithelial cell infection model is 0.30 µg/mL, indicating that the monoclonal antibody Fab5 can significantly inhibit EBV infection of epithelial cells.

### Example 5: Neutralization activity assay of Fab5 monoclonal antibody on B cells

1) Fab5 monoclonal antibody from the above example was adjusted to a concentration of 2 mg/mL. In a new 96-well plate, 60 µL of RPMI1640 medium was added to each well. 90 µL of 100 µg/mL Fab5 antibody diluted with RPMI1640 was added to the first well (the first well did not contain RPMI1640 medium).
2) After 3-fold serial dilution (the serial dilution procedure was as follows: 30 µL was aspirated from the first well and added to the second well, and so on; 30 µL was aspirated from the last well and discarded; a final volume per well was 60 µL), 60 µL of virus diluent (the virus was diluted with DMEM medium to a titer of approximately 4×10⁶/mL) was added to each well, incubated at 37 °C for 2 hours, and then 1×10⁶ Raji cells were added per well. The cells were placed in a 37 °C incubator and cultured for 48 hours for the assay.
3) The Raji cells were aspirated to prepare a cell suspension. The infection rate was detected by flow cytometry. By measuring the reduction ratio in the number of GFP-positive cells in the antibody-treated group compared to the infection control group (to which an equal volume of RPMI1640 was added), the inhibition rate (neutralization efficiency, %) of the antibody in the Raji B cell infection model was calculated. By using Prism software for plotting and calculation, the IC₅₀ of the Fab5 monoclonal antibody was obtained.

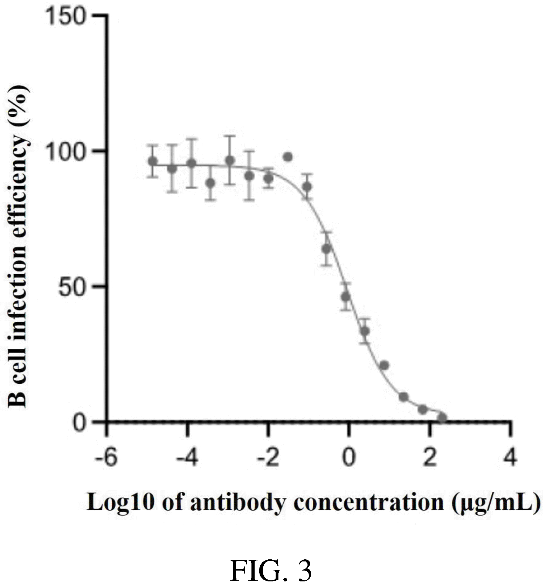

The results are shown in FIG. 3: the IC₅₀ of the monoclonal antibody Fab5 in the B cell infection model is 0.9 µg/mL, indicating that the monoclonal antibody Fab5 can significantly inhibit EBV infection of B cells.

### Example 6: Cryo-Electron Microscopy (Cryo-EM) structure of monoclonal antibody Fab5 in complex with gB protein

Brief steps for cryo-electron microscopy imaging were as follows:
(1) 1 mg/mL Fab5/gB complex sample was dropped at a molar ratio of 1:1 (M/M) onto a glow-discharged Quantifoil Cu R1.2/1.3 300-mesh copper grid.
(2) Cryo-sample preparation was performed using an automatic cryo-sample preparation robot, Vitrobot Mark IV, with a freezing time of 7 seconds and a humidity of 100%.
(3) The sample was observed using a 300 kV Titan Krios transmission electron microscope. If the sample preparation was unqualified, cryo-sample preparation was repeated.
(4) Qualified samples were imaged using a K2 Summit direct detection device, and the original images were recorded.

Brief steps for cryo-EM single-particle analysis were as follows:
(1) The imaging data were aligned using motionCor2.
(2) The contrast transfer function (CTF) values of the cryo-EM data were calculated using PatchCTF.
(3) Automatic and reference-free particle picking was performed using Blob Picker to generate 2D classification reference images.
(4) Based on the best set of reference images, reference density was further selected for reference-based particle picking and 2D classification.
(5) Homogeneous reconstruction was performed on the particles from the best set of 2D classes, and the resolution was determined according to the gsFSC standard.

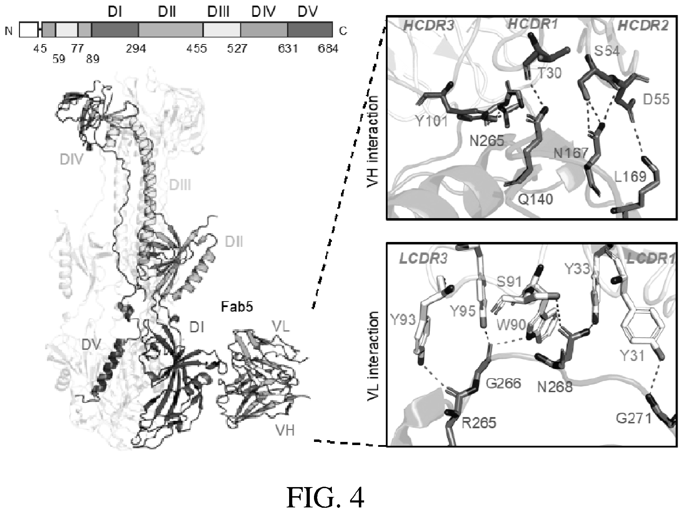

The results are shown in FIG. 4. The structure shows that Fab5 binds to the DI domain of the gB protein, wherein the heavy chain of Fab5 is shown as medium-dark gray and the light chain as lighter gray. Through further binding interface analysis, precise information on the key interaction regions of the heavy and light chains with gB was obtained. Moreover, the feature that Fab5 binds to the DI region is significantly different from previously reported antibodies 3A3 and 3A5 (Zhang et al. PNAS, 2022), highlighting that Fab5 is a novel Epstein-Barr virus neutralizing antibody. The structure of the Fab5-gB protein complex provides important molecular biological evidence for the significant gB affinity and high EBV neutralization activity of antibody Fab5.

## Claims

1. A monoclonal antibody or an antigen-binding fragment thereof against EBV gB protein, wherein the monoclonal antibody or the antigen-binding fragment thereof comprises a heavy chain variable region and a light chain variable region;
the heavy chain variable region comprises CDR-H1, CDR-H2, and CDR-H3;
the CDR-H1, CDR-H2, and CDR-H3 are CDR1, CDR2, CDR3 of an amino acid sequence shown in SEQ ID NO: 4;
the light chain variable region comprises CDR-L1, CDR-L2, and CDR-L3;
the CDR-L1, CDR-L2, and CDR-L3 are CDR1, CDR2, CDR3 of an amino acid sequence shown in SEQ ID NO: 17.

2. The monoclonal antibody or the antigen-binding fragment thereof according to claim 1, wherein:
amino acid sequences of the CDR-H1, CDR-H2, CDR-H3, CDR-L1, CDR-L3 are sequentially as shown in SEQ ID NO: 5, SEQ ID NO: 6, SEQ ID NO: 7, SEQ ID NO: 18, and SEQ ID NO: 19, an amino acid sequence of the CDR-L2 is DTS, and the CDRs are defined by an IMGT definition scheme; or
the amino acid sequences of the CDR-H1, CDR-H2, CDR-H3, CDR-L1, CDR-L2, CDR-L3 are sequentially as shown in SEQ ID NO: 8, SEQ ID NO: 9, SEQ ID NO: 10, SEQ ID NO: 20, SEQ ID NO: 21, and SEQ ID NO: 19, and the CDRs are defined by a Kabat definition scheme; or
the amino acid sequences of the CDR-H1, CDR-H2, CDR-H3, CDR-L1, CDR-L2, CDR-L3 are sequentially as shown in SEQ ID NO: 11, SEQ ID NO: 12, SEQ ID NO: 10, SEQ ID NO: 20, SEQ ID NO: 21, and SEQ ID NO: 19, and the CDRs are defined by a Chothia definition scheme; or
the amino acid sequences of the CDR-H1, CDR-H2, CDR-H3, CDR-L1, CDR-L2, CDR-L3 are sequentially as shown in SEQ ID NO: 13, SEQ ID NO: 14, SEQ ID NO: 15, SEQ ID NO: 22, SEQ ID NO: 23, and SEQ ID NO: 24, and the CDRs are defined by a Contact definition scheme.

3. The monoclonal antibody or the antigen-binding fragment thereof according to claim 1 or 2, wherein the monoclonal antibody or the antigen-binding fragment thereof comprises at least one of a full-length antibody, Fab, Fab', F(ab')2, Fv, scFv, a bispecific antibody, or a multispecific antibody.

4. A recombinant protein, comprising the monoclonal antibody or the antigen-binding fragment thereof according to any one of claims 1-3; and optionally a tag sequence that assists expression and/or purification.

5. A biological material related to the monoclonal antibody or the antigen-binding fragment thereof according to any one of claims 1-3, or the recombinant protein according to claim 4, wherein the biological material comprises at least one of h1) to h16):
h1) a nucleic acid molecule encoding the monoclonal antibody or the antigen-binding fragment thereof according to any one of claims 1-3, or the recombinant protein according to claim 4;
h2) an expression cassette comprising the nucleic acid molecule of h1);
h3) a vector comprising the nucleic acid molecule of h1);
h4) a vector comprising the expression cassette of h2);
h5) a transgenic cell line comprising the nucleic acid molecule of h1);
h6) a transgenic cell line comprising the expression cassette of h2);
h7) a transgenic cell line comprising the vector of h3);
h8) a transgenic cell line comprising the vector of h4);
h9) a microorganism comprising the nucleic acid molecule of h1);
h10) a microorganism comprising the expression cassette of h2);
h11) a microorganism comprising the vector of h3);
h12) a microorganism comprising the vector of h4);
h13) a virus comprising the nucleic acid molecule of h1);
h14) a virus comprising the expression cassette of h2);
h15) a virus comprising the vector of h3);
h16) a virus comprising the vector of h4).

6. A method for preparing the monoclonal antibody or the antigen-binding fragment thereof according to any one of claims 1-3, or the recombinant protein according to claim 4, comprising the following steps:
expressing and then purifying the biological material according to claim 5 to obtain the monoclonal antibody or the antigen-binding fragment thereof or the recombinant protein.

7. A conjugate comprising at least one of the monoclonal antibody or the antigen-binding fragment thereof according to any one of claims 1-3, or the recombinant protein according to claim 4; and
a conjugated moiety, wherein the conjugated moiety comprises at least one of a detectable label, a drug, a toxin, a cytokine, a radionuclide, or an enzyme.

8. Use of at least one of (1) to (4) in the preparation of a product:
(1) the monoclonal antibody or the antigen-binding fragment thereof according to any one of claims 1-3;
(2) the recombinant protein according to claim 4;
(3) the biological material according to claim 5;
(4) the conjugate according to claim 7;
wherein the product comprises at least one of a drug, a vaccine, a reagent, a detection plate, a kit, or a detection chip.

9. A drug, comprising at least one of (l1) to (l4):
(l1) the monoclonal antibody or the antigen-binding fragment thereof according to any one of claims 1-3;
(l2) the recombinant protein according to claim 4;
(l3) the biological material according to claim 5;
(l4) the conjugate according to claim 7;
preferably, the drug further comprises a pharmaceutically acceptable carrier.

10. A kit for detecting Epstein-Barr virus, wherein the kit comprises at least one of (l1) to (l4) and an adjuvant:
(l1) the monoclonal antibody or the antigen-binding fragment thereof according to any one of claims 1-3;
(l2) the recombinant protein according to claim 4;
(l3) the biological material according to claim 5;
(l4) the conjugate according to claim 7.
